Europäisches Patentamt

(19) European Patent Office

Office européen des brevets

(11) Publication number: **0 118 549**

**B1**

## EUROPEAN PATENT SPECIFICATION

(45) Date of publication of patent specification: **13.12.89**   (51) Int. Cl.⁴: **A 61 F 2/24**

(21) Application number: **83903061.6**

(22) Date of filing: **06.09.83**

(86) International application number:
**PCT/US83/01368**

(87) International publication number:
**WO 84/00882 15.03.84 Gazette 84/07**

(54) **PROSTHETIC HEART VALVE WITH PLANO-CONVEX DISK OCCLUDER.**

(30) Priority: **07.09.82 US 415283**

(43) Date of publication of application:
**19.09.84 Bulletin 84/38**

(45) Publication of the grant of the patent:
**13.12.89 Bulletin 89/50**

(84) Designated Contracting States:
**AT BE CH DE FR GB LI NL SE**

(56) References cited:
**GB-A-2 051 308**
**US-A-4 240 161**
**US-A-4 343 049**

(73) Proprietor: **ANGICOR LIMITED**
**15301 Highway 55 West**
**Minneapolis MN 55447 (US)**

(72) Inventor: **KASTER, Robert, L.**
**2730 Vagabond Lane**
**Plymouth, MN 55447 (US)**

(74) Representative: **SERJEANTS**
**25, The Crescent King Street**
**Leicester, LE1 6RX (GB)**

Courier Press, Leamington Spa, England.

## Description

Technical Field

This invention relates generally to artificial heart valves, and more particularly to heart valves that utilize a free-floating, rotatable, pivotable disk occluder.

Background Art

The human heart serves as a four chambered double pump. Four separate valves control the passage of blood between these chambers. When functioning properly, these natural valves operate as one way mechanisms that allow blood to flow in only one direction therethrough. When one of these valves operates defectively, serious health concerns arise. Often the defective valve must be replaced with a prosthetic device.

Artificial heart valves are utilized for the above purpose. Typically, such valves include a circular orifice and a flow regulating device such as a ball or disk that may be used to occlude the orifice passageway. Examples of such heart valves are disclosed in GB 2051308A, US 4240161 and US 4343049, which are discussed below.

Such implanted heart valves must withstand a number of stressful conditions and are otherwise subject to an unfavourable environment for artifacts. For instance, large static and dynamic inertias are evident as the heart pumps blood. Furthermore, a significant hemodynamic pressure gradient may be built up across the valve location.

Heart valves are also subject to localized wear and breakdown, and can contribute to the creation of stagnation areas where clotting may occur.

Ideally, an artificial heart valve provides minimal opposition to the desired flow of blood and additionally encourages a centralized flow through the valve. Heart valves that make use of free floating, rotatable, pivotable disk occluders seem to meet these criteria best. Nevertheless, prior art devices that make use of such disk occluders still have not insignificant problems in accommodating the hemodynamic pressure gradient and in minimizing the creation of stagnation areas. Further, such disk occluders have not been as successful in encouraging a centralized flow of blood as might be otherwise desired.

GB 2051308A, US 4240161 and US 4343049 each discloses a prosthetic heart valve comprising a mounting ring mounting a disk occluder in a free-floating and pivotable manner to permit pivotal movement of the occluder between occlude and non-occlude positions. Guide means are provided around the rim of the mounting ring for supporting the disk occluder and guiding and limiting its pivotal movement. GB 2051308A discloses, on the downstream side of the disk occluder, a re-entrant recess which receives the guide means which is a shaped bar projecting from the rim of the mounting ring. The re-entrant shape of the recess is important in retaining the disk occluder in position and in controlling its pivotal movement. US 4343049 also discloses a recess on the downstream side of the disk occluder, used as a bearing surface for the guide means. In US 4343049 the recess is not re-entrant, and so a further guide member is necessary spanning a major cord of the mounting disk and positioned immediately beneath the guide means in the recess, as a means for retaining the disk occluder in position.

US 4240161, on the other hand, discloses a disk occluder which is mounted wholly around its rim. The mounting is in no way dependent on provision of a guide bar across the downstream side of the disk occluder, so no well or recess is needed on the downstream side of the disk, in which to locate such a guide bar. Instead, the disk is disclosed as being generally convex on its downstream side and concave on its upstream side, giving the advantage that it is self-cleaning in use under a pulsating flow of blood. Unfortunately however the design is not so robust or reliable as that of the other two prior art documents, as a consequence of the rim mounting of the disk.

Disclosure of the Invention

The above problems are substantially resolved, without undue compromise of other desirable attributes noted above that are already provided by prior art devices, by provision of the invention disclosed herein. The invention includes generally a valve housing and a free floating, rotatable, pivotable disk occluder.

The disk occluder includes a substantially planar proximal surface and a substantially convex distal surface. (Throughout this specification and in the claims, the words "proximal" and "distal" are not used in their usual euclidean connotation. Rather, in cardiovascular practice, "proximal" refers to that place where blood enters a particular structure, and "distal" refers to that place where blood leaves a particular structure. Therefore, as used in reference to a disk occluder, "proximal" refers to that side of the disk occluder that normally faces the blood inflow port and the word "distal" refers to that side of the disk occluder that normally faces the blood outflow port.)

The substantially planar proximal surface of the disk occluder includes a well concentrically disposed therein that cooperates with a complementary guide means in the form of a pivot and support structure that will be described further below. Finally, the disk occluder preferably includes a rounded or curved periphery that smoothly joins the substantially convex distal surface with the substantially planar proximal surface.

The valve housing of the invention may be comprised generally of an annularly shaped base. This base includes a suture ring groove formed about the exterior periphery thereof for receiving a sewing ring to facilitate implantation of the valve within the heart. In addition, the inner circumference of the base may be rounded. The valve housing also includes a distal positioned control unit and a proximal positioned control unit.

The proximal positioned control unit may be comprised of a cantilevered structure that provides both an occlude mode support surface and a first non-occlude mode support surface that operates cooperatively with the well disposed on the disk occluder. (as used herein, a "support surface" includes a planar surface, line surface or point surface, without limitation.) The occlude mode support surface cooperates with both the well of the disk occluder and the planar surface thereof. In addition, the proximal positioned control unit provides a non-occlude to occlude mode transition pivot guide.

The proximal positioned control unit may be formed of a rod like structure that will sturdily support the pivot motion, occluding position and non-occluding position of the disk occluder while simultaneously presenting only a minimum barrier to the flow of blood.

The distal positioned control unit serves both as an occlude to non-occlude mode transition pivot guide and as a second non-occlude mode support surface. The distal positioned control unit may be provided by the use of two short projections that are located opposite one another along a cord of the valve housing. These projections are oriented substantially inwardly of the valve housing and cooperate with the convex surface of the disk occluder such that the disk occluder may pivot with respect to the projections and may further be supported thereby when in the non-occlude mode.

When blood pressure on the proximal side of the disk occluder exceeds the pressure on the distal side, the disk occluder will translate slightly distally and then pivot about the pivot guide surfaces of the distal positioned control unit. The disk occluder will come to rest against the support surfaces described above at approximately a 75° angle with the horizontal, and can move no further.

So positioned, two blood flow apertures are formed; one aperture being larger than the other. The larger aperture becomes formed between the valve housing and the proximal side of the disk occluder. The smaller aperture becomes formed between the valve housing and the distal side of the disk occluder.

The convex surface of the disk occluder cooperates with the planar surface of the disk occluder, when positioned in the non-occlude mode, to non-turbulently divide the flow of blood between the smaller and larger, apertures. A sufficient quantity of blood will pass non-turbulently through the smaller aperture such that the blood will be substantially centrally directed through the valve housing. The rounded edge of the disk occluder further enhances the non-turbulent passage of blood.

As a result, a heart valve utilizing this disk occluder well imitates the functioning of a natural heart valve with respect to the maintenance of a substantially centralized flow of blood.

Brief Description of the Drawings

These and other attributes of the invention will become more clear upon a thorough study and review of the following detailed description of the best mode for carrying out the invention, particularly when reviewed in conjunction with the drawings, wherein:

Fig. 1 is a top plan view of the heart valve with the disk occluder in the occlude mode;

Fig. 2 is a top plan view of the heart valve with the disk occluder removed;

Fig. 3 is a top plan view of the heart valve with the disk occluder in the non-occlude mode;

Fig. 4 is a front elevational view of the invention sectioned along the line 4-4 as depicted in Fig. 3;

Fig. 5 is a side elevational view of the invention sectioned along the line 5-5 as depicted in Fig. 3;

Fig. 6 is a detail sectioned side elevational view of the invention;

Fig. 7 is a sectioned detail view taken along the line 7-7 as depicted in Fig. 5;

Fig. 8 is a side elevational sectioned view of the disk occluder of the invention; and

Fig. 9 is a top plan view of the disk occluder.

Best Mode for Carrying Out the Invention

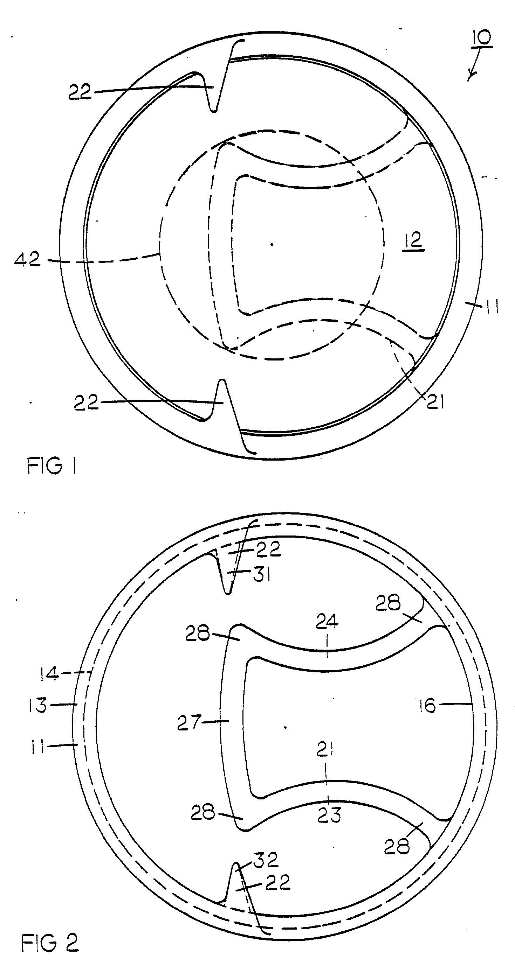

Referring now to the drawings, and in particular to Fig. 1, the heart valve of the invention may be seen as depicted generally by the numeral 10. The heart valve (10) includes generally a valve housing (11) and a disk occluder (12). These general elements will now be described in seriatim fashion.

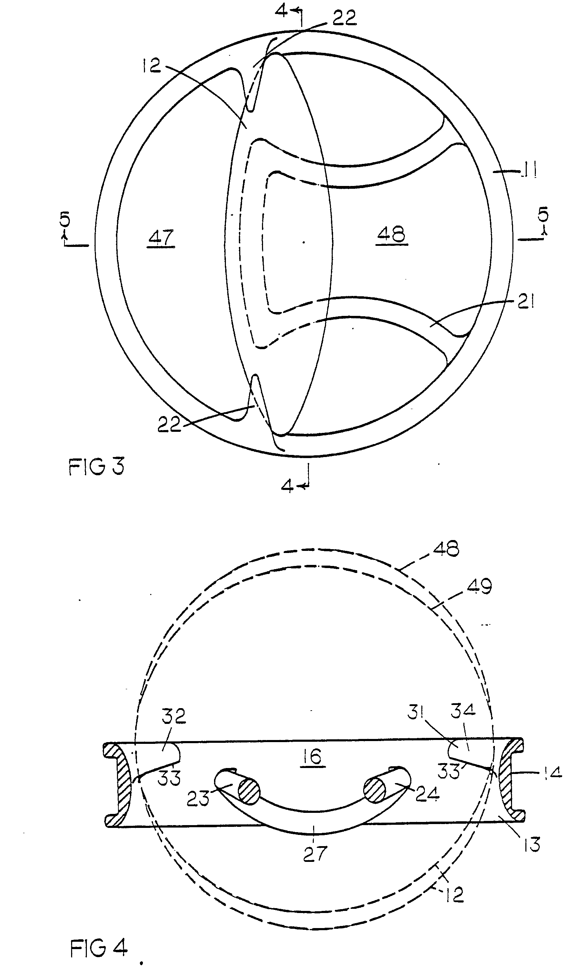

Referring now to Fig. 2, the valve housing (11) will be described in detail. The valve housing (11) includes an annularly shaped base (13). This annularly shaped base (13) includes a suture ring groove (14) that is perhaps best depicted at Fig. 4. A sewing ring (not depicted may be disposed within the suture ring groove (14) to facilitate implantation of the heart valve (10) within a heart (not shown). Such sewing rings are well known in the art. Therefore, no further detailed disclosure of such sewing rings need be made here.

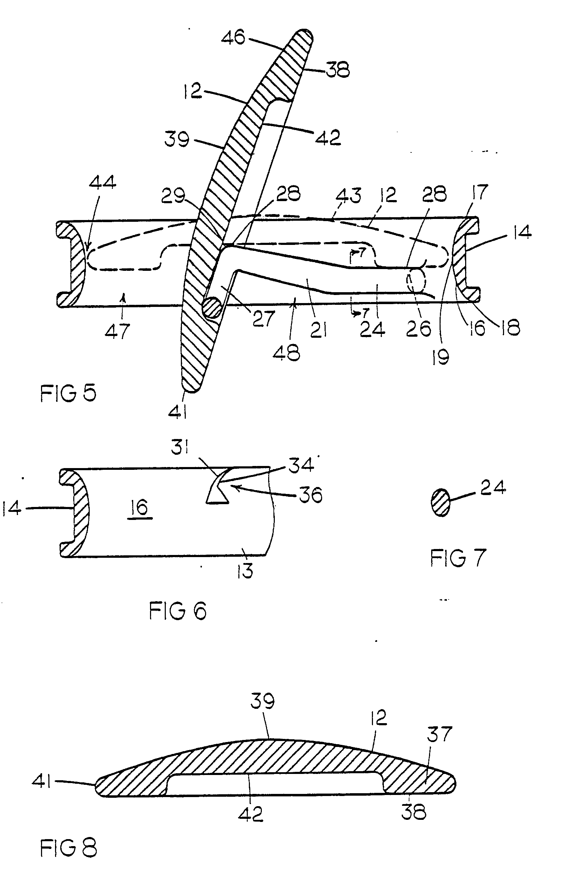

With reference to Fig. 5, the interior circumferential surface (16) thereof may be crowned. More particularly, the interior surface (16) may widen in circumference at both edges (17 and 18) and be narrowest at the center (19). It may further be noted that the interior circumference at the edges (17 and 18) may be greater than the diameter of the suture ring groove (14). Such an arrangement will assist in assuring that the valve housing (11) obstructs the flow of blood therethrough as little as possible when implanted within a heart.

The valve housing (11) also includes an integrally formed distal positioned control unit (22) and a proximal positioned control unit (21). Both of these units (21 and 22) will now be described with reference to Fig. 2.

The proximal positioned control unit (21) attaches to the interior surface (16) of the valve housing (11). This connection may be accomplished through two curved rod like members (23 and 24) that are affixed to the proximal portion of the base (13) (see Fig. 5 at numeral 26). As represented in Fig. 7, both rod like members (23 and 24) have a generally elliptical cross section.

Both rod like members (23 and 24) are cantilevered towards the interior of the valve housing (11). Both rod like members (23 and 24) are then joined at their cantilevered ends by a generally U-shaped member (27), the U-shaped member (27) being perhaps best portrayed in Fig. 4.

A section of the distal surface of both ends of each rod like member (23 and 24) serves as an support surface for the disk occluder (12). These areas of support are depicted generally by the numeral 28. Both cantilevered ends of the rod-like members (23 and 24) serve as a pivot guide for the disk occluder during the transition from the non-occluding mode to the occlude mode, these pivot guide areas being represented generally by the numeral 29.

The U-shaped member (27) serves as a non-occlude mode support surface as depicted in Fig. 5. A detailed description of cooperative functioning between the disk occluder and the proximal positioned control unit (21) will be provided below.

Preferably, the proximal positioned control unit (21) will be fashioned such that the valve housing base (13), the two rod like members (23 and 24) and the U-shaped member (27) will all be integrally formed.

The distal positioned control unit (22) may be comprised of two substantially triangularly shaped projections (31 and 32). Since each projection (31 and 32) constitutes a mirror image of the other, only one such projection (32) need be described in detail.

The projection (32) attaches to the distal portion of the interior surface (16) of the base (13). As depicted in Figs. 4 and 6, the underside surface (33) of the projection (31) angles upwardly somewhat from the horizontal plane. The inwardly disposed surface (34) of the projection (31) has a more arcuate shape. It may be seen that the inwardly disposed surface (34) curves to form a concave receiving area for operable cooperation with the convex surface of the disk occluder (12).

The distal positioned control unit (22) serves as a pivot guide for the disk occluder (12) during the transition from the occlude mode to the non-occlude mode. Second, the distal positioned control unit (22) serves as a support surface for the disk occluder (12) during the non-occlude mode. As with the proximal positioned control unit (21), the means of cooperation between the distal position control unit (22) and the disk occluder (12) will be described below.

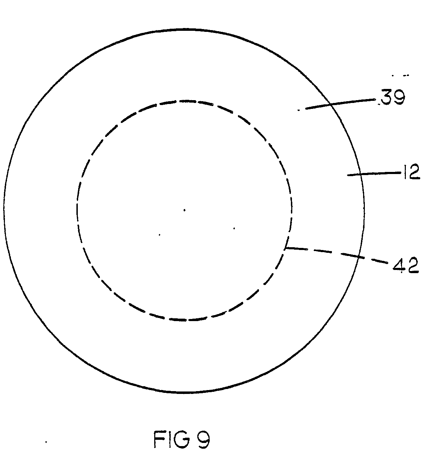

With reference to Figs. 8 and 9, the disk occluder (12) comprises a substantially circular disk (37). This disk (37) has a substantially planar underside surface (38) and a substantially convex upper surface (39). The substantially planar under surface (38) will henceforth be referred to as the distal surface, and the substantially convex upper surface of the disk (37) will be referred to as the distal surface. The periphery of the disk (37) may be rounded (41) for reasons that are explained below. Finally, a well (42) may be concentrically disposed on the proximal surface of the disk (37) for cooperative interaction with the proximal positioned control unit (21).

Both the valve housing (11) and the disk occluder (12) should be formed of materials that are compatible with the host environment. In addition, the materials should preferably be radiopaque and be lightweight. Such materials could include Pyrolite®-carbon, sapphire, ceramic, metals (such as titanium, tantalum, and stainless steel) and plastics (such as polypropylene, polycarbonate, polysulfone, polyethelene, Delrin®, Teflon® or the like). The applicant prefers the use of Pyrolite®-carbon for the disk occluder and titanium for the valve housing (11).

The heart valve (10) may be constructed or formed by any known means that is coincidentally suitable for the material being used. Such means of manufacture include machining, casting, molding, welding, swagging, electron discharge machining (EDM), stamping or the like. For this particular heart valve, the applicant prefers a machining, method.

Operation of the heart valve (10) will now be described. With reference to Figs. 1 and 5, the disk occluder (12) may be seen in the occlude mode (in Fig. 5 the occlude mode is represented by phantom lines (43)). It will be appreciated that the base (13) does not include a circumferentially disposed seat or the like for interacting with the disk occluder (12). Rather, a small clearance on the order of .1 mm. (44) may be provided between the disk occluder periphery (41) and the crown (19) of the interior surface (16) of the valve housing base (13).

Rather than being supported by such a seat, the disk occluder (12) rests upon four different sections of the proximal positioned control unit (21). Such support has been provided near the base and terminus of the proximal positioned control unit (21) as represented by the numeral 28.

When in the occlude mode, the proximal positioned control unit (21) (Fig. 5) cooperatively interacts with the well (42) provided in the disk occluder (12). This cooperation assures that the disk occluder (12) may rotate about its axis as may be necessary, but the disk occluder will yet be prevented from unwanted lateral movement.

When blood pressure on the proximal side of the disk occluder increases and exceeds the blood pressure on the distal side of the disk occluder, the disk occluder (12) will begin the transition from the occlude mode (as depicted by the numeral 43) to the non-occlude mode (as depicted by the numeral 46). This transition begins with a small distal translation of the disk occluder (12) relative the valve housing (11). This translation may only continue until the distal surface (39) of the disk occluder (12) contacts the distal positioned control unit (22). Once such contact has been made, translation will substantially cease and pivoting will begin.

The disk occluder (12) will pivot about the projections (31 and 32) of the distal position control unit (22) until the disk occluder (12) contacts the non-occlude mode support surface (36) of the distal positioned control unit (22) and/or the non-occlude mode support surface represented by the U-shaped member (27) of the proximal positioned control unit (21). As such pivoting occurs, the disk occluder (12) will also be able to translate further in the distal direction, and such further translation does occur.

So disposed, the proximal surface (38) of the disk occluder (12) will be disposed at an angle of approximately 75° with respect to the horizontal. Other angular positions could be utilized as well, of course, within a range of approximately 50° to 89°.

It will be appreciated that when so positioned, the substantially planar distal surface (38), the rounded periphery (41) and the substantially convex distal surface (39) of the disk occluder (12) cooperate to divide and channel the blood flow as between two apertures that have been formed in the valve housing opening (11) by the position of the disk occluder (12) with minimum obstruction.

In this respect, it may be noted that the disk occluder (12) does not pivot about a centrally disposed axis. Rather, the pivoting occurs off center. Therefore, the aperture formed between the valve housing (11) and the distal portion of the disk occluder (12) constitutes a smaller aperture (47) than the aperture (48) formed between the valve housing (11) and the proximal side (38) of the disk occluder (12).

The shape and position of the disk occluder (12) assures that blood will be directed through both the smaller and larger apertures (47 and 48) to promote a substantially centralized flow of blood through the valve housing (11).

When pressure on the distal side of the valve housing (11) begins to exceed that on the proximal side, the disk occluder (12) will begin the transition from the non-occlude mode (46) to the occlude mode (43). This transition begins with a small proximal translation of the disk occluder (12). This translation has been depicted by the use of phantom lines in Fig. 4, wherein the numeral 48 refers to the distal position of the disk occluder (12), and the numeral 49 refers to the proximal position thereof.

The disk occluder (12) continues this translation to the proximal position (49) until the periphery of the disk occluder (41) contacts the interior surface (16) of the base (13). When such contact has been made, the disk occluder will pivot about the pivot surface represented by the numeral 29 on the proximal positioned control unit (21). Such pivoting will continue until the disk occluder (12) assumes the complete occlude mode position (43).

It will be appreciated that the heart valve (10) of the invention achieves all of the benefits of earlier free floating, rotatable, pivotable disk occluder heart valves, and that the heart valve (10) of the invention achieves further advantages as well.

Most importantly, this heart valve (10) assures a more centralized and non-turbulent flow of blood through the heart valve (10) with all of its attendant advantages.

Other advantages are obtained as well. For instance, the proximal positioned control unit (21) cooperates with the inner surface of the concentrically disposed well (42) to aid in preventing the disk (37) from moving further in the distal direction when in the non-occlude position. Also, disk (37) can freely rotate about the proximal positioned control unit (21). As a result, biologic deposits such as coagulated blood can be continually washed from the proximal surface of the disk (37), including the concentrically disposed well (42), by interaction between these two parts.

Obviously, many modifications and variations of the present invention are possible in light of the above teachings. It is therefore to be understood that, within the scope of the appended Claims, the invention may be practised otherwise than as specifically described.

## Claims

1. A prosthetic heart valve comprising a mounting ring (11) mounting a disk occluder (12) in a free-floating, rotatable and pivotable manner to permit pivotal movement of the occluder (12) between an occlude position preventing blood flow and a non-occlude position permitting the flow of blood from a proximal inlet side of the heart valve to a distal outlet side and proximal positioned and distal positioned guide means (21, 22) for operative interaction with the disk occluder (12) for supporting the disk occluder (12) and guiding and limiting its pivotal movement, characterised in that the disk occluder (12) comprises a substantially planar proximal surface (38) in which is formed a well (42) which provides a bearing surface for at least a part of the proximal positioned guide means (21) during at least a part of the pivotal movement of the disk occluder (12) from the occlude position to the non-occlude position.

2. A prosthetic heart valve according to claim 1, wherein the guide means (21, 22) are such that the disk occluder (12) is at least partially supported by the proximal positioned guide means (21) when in the occlude position and is at least partially supported by the distal positioned guide means (22) when in the non-occlude position.

3. A prosthetic heart valve according to claim 2, wherein the disk occluder is also partially supported by the proximal positioned guide means (21) when in the non-occlude position.

4. A prosthetic heart valve according to any preceding claim wherein the proximal positioned guide means (21) comprises a pair of rod members (23, 24) each extending in cantilever from the mounting ring (11) inwardly of the mounting ring.

5. A prosthetic heart valve according to claim 4, wherein the rod members (23, 24) lie generally in the plane of the mounting ring.

6. A prosthetic heart valve according to claim 4 or claim 5, wherein the proximal positioned guide means (21) further comprises a generally arcuate member (27) mounted between the inner ends of the rod members (23, 24) and proportioned and positioned to lie within the well (42) in contact with the bearing surface of the well (42) immediately adjacent a side wall of the well (42) throughout the length of the arc when the disk occluder (12) is in the non-occluded position.

7. A prosthetic heart valve according to claim 6, wherein the arcuate member (27) is in a plane aligned at an angle of from 50° to 89° to the plane of the mounting ring (11).

8. A prosthetic heart valve according to claim 7, wherein the arcuate member (27) is in a plane aligned at an angle of substantially 75° to the plane of the mounting ring (11).

9. A prosthetic heart valve according to any preceding claim, wherein the shape and dimensions of the well (42) and of the proximal positioned guide means (21) are mutually complementary to aid the prevention of the accumulation of unwanted biologic material within the well (42) in use.

10. A prosthetic heart valve according to any preceding claim, wherein the distal positioned guide means (22) comprises first and second substantially triangularly shaped projections (31, 32), each extending inwardly of the mounting ring (11) and each having a bearing surface (34) for cooperation with the distal surface of the disk occluder (12).

**Patentansprüche**

1. Prothetische Herzklappe bzw. -ventil mit einem Befestigungsring (11), an dem eine Verschlußscheibe (12) schwimmend, drehbar und schwenkbar angeordnet ist, um eine Schwenkbewegung der Verschlußscheibe (12) zwischen einer Geschlossenstellung, die den Blutfluß verhindert, und einer Nicht-Geschlossenstellung, die den Blutfluß von einer proximalen Einlaßseite der Herzklappe zu einer distalen Auslaßseite ermöglicht, und mit proximal und distal angeordneten Führungen (21, 22) zum operativen Zusammenwirken mit der Verschlußscheibe (12), wobei die Verschlußscheibe (12) abgestützt, geführt und ihre Schwenkbewegung begrenzt wird, dadurch gekennzeichnet, daß die Verschlußscheibe (12) eine im wesentlichen ebene proximale Oberfläche (38) aufweist, in welcher eine Vertiefung (42) ausgebildet ist, welche eine Anlagefläche für zumindest einen Teil der proximal angeordneten Führung (21) während mindestens eines Abschnittes der Schwenkbewegung der Verschlußscheibe (12) von der Geschlossenstellung in die Nicht-Geschlossenstellung umfaßt.

2. Prothetische Herzklappe bzw. -ventil nach Anspruch 1, dadurch gekennzeichnet, daß die Führungen (21, 22) derart ausgebildet sind, daß die Verschlußscheibe (12) zumindest teilweise durch die proximal angeordnete Führung (21) abgestützt ist, wenn sie sich in der Geschlossenstellung befindet, und zumindest teilweise durch die distal angeordnete Führung (22) abgestützt ist, wenn sie sich in der Nicht-Geschlossenstellung befindet.

3. Prothetische Herzklappe bzw. -ventil nach Anspruch 2, dadurch gekennzeichnet, daß die Verschlußscheibe (12) teilweise auch durch die proximal angeordnete Führung (21) abgestützt ist, wenn sie sich in der Nicht-Geschlossenstellung befindet.

4. Prothetische Herzklappe bzw. -ventil nach irgendeinem der vorhergehenden Ansprüche, dadurch gekennzeichnet, daß die proximal angeordnete Führung (21) ein Paar Stäbe (23, 24) umfaßt, von denen sich jeder derart erstreckt, daß er von dem Befestigungsring (11) in das Innere des Befestigungsringes auskragt.

5. Prothetische Herzklappe bzw, -ventil nach Anspruch 4, dadurch gekennzeichnet, daß die Stäbe (23, 24) im allgemeinen in der Ebene des Befestigungsringes liegen.

6. Prothetische Herzklappe bzw. -ventil nach Anspruch 4 oder 5, dadurch gekennzeichnet, daß die proximal angeordnete Führung (21) außerdem einen im allgemeinen bogenförmigen Abschnitt (27) umfaßt, der zwischen den inneren Enden der Stäbe (23, 24) befestigt und derart gestaltet und angeordnet ist, daß er innerhalb der Vertiefung (42) in Kontakt mit der Anlagefläche der Vertiefung (42) über die gesamte Länge des Bogens unmittelbar anschließend an eine Seitenwand der Vertiefung (42) ist, wenn die Verschlußscheibe (12) sich in der Nicht-Geschlossenstellung befindet.

7. Prothetische Herzklappe bzw. -ventil nach Anspruch 6, dadurch gekennzeichnet, daß sich der bogenförmige Abschnitt (27) in einer Ebene erstreckt, die mit der Ebene des Befestigungsringes (11) einen Winkel von 50° bis 89° einschließt.

8. Prothetische Herzklappe bzw. -ventil nach Anspruch 7, dadurch gekennzeichnet, daß sich der bogenförmige Abschnitt (27) in einer Ebene erstreckt, die mit der Ebene des Befestigungsringes (11) einen Winkel von im wesentlichen 75° einschließt.

9. Prothetische Herzklappe bzw. -ventil nach irgendeinem der vorhergehenden Ansprüche, dadurch gekennzeichnet, daß die Form und die Abmessungen der Vertiefung (42) und der proximal angeordneten Führung (21) einander komplementär sind, um das Verhindern der Anhäufung von unerwünschtem biologischen Material innerhalb der Vertiefung (42) beim Gebrauch zu unterstützen.

10. Prothetische Herzklappe bzw. -ventil nach irgendeinem der vorhergehenden Ansprüche, dadurch gekennzeichnet, daß die distal angeordnete Führung (22) erste und zweite, im wesentlichen dreieckig geformte Vorsprünge (31, 32) umfaßt, von welchen sich jeder vom Befestigungsring (11) nach innen erstreckt und von welchen jeder eine Anlagefläche (34) für das Zusammenwirken mit der distalen Oberfläche der Verschlußscheibe (12) aufweist.

## Revendications

1. Valvule cardiaque prothétique comprenant une bague (11) de montage portant un disque obturateur (12) d'une manière flottant librement, pouvant tourner et pivoter afin de permettre un mouvement de pivotement de l'obturateur (12) entre une position d'obturation empêchant l'écoulement du sang et une position de non-obturation permettant l'écoulement du sang d'un côté proximal d'entrée de la valvule cardiaque vers un côté distal de sortie et des moyens de guidage (21, 22) en position proximale et en position distale pour une interaction fonctionnelle avec le disque obturateur (12) afin de supporter le disque obturateur (12) et d'en guider et limiter le mouvement de pivotement, caractérisé en ce que le disque obturateur (12) comprend une surface proximale sensiblement plane (38) dans laquelle est formé un alvéole (42) qui présente une surface d'appui pour au moins une partie du moyen de guidage (21) en position proximale pendant au moins une partie du mouvement de pivotement du disque obturateur (12) de la position d'obturation vers la position de non-obturation.

2. Valvule cardiaque prothétique selon la revendication 1, dans laquelle les moyens de guidage (21, 22) sont tels que le disque obturateur (12) est au moins partiellement supporté par le moyen de guidage (21) en position proximale lorsqu'il se trouve dans la position d'obturation et est au moins partiellement supporté par le moyen de guidage (22) en position distale lorsqu'il se trouve dans la position de non-obturation.

3. Valvule cardiaque prothétique selon la revendication 2, dans laquelle le disque obturateur est également partiellement supporté par le moyen de guidage (21) en position proximale lorsqu'il se trouve dans la position de non-obturation.

4. Valvule cardiaque prothétique selon l'une quelconque des revendications précédentes, dans laquelle le moyen de guidage (21) en position proximale comprend deux tiges (23, 24) s'étendant chacune en porte-à-faux à partir de la bague (11) de montage vers l'intérieur de cette bague de montage.

5. Valvule cardiaque prothétique selon la revendication 4, dans laquelle les tiges (23, 24) s'étendent globalement dans le plan de la bague de montage.

6. Valvule cardiaque prothétique selon la revendication 4 ou la revendication 5, dans laquelle le moyen de guidage (21) en position proximale comprend en outre un élément globalement incurvé (27) monté entre les extrémités intérieures des tiges (23, 24) et proportionné et positionné de façon à s'étendre à l'intérieur de l'alvéole (42) en contact avec la surface d'appui de l'alvéole (42) à proximité immédiate d'une paroi latérale de l'alvéole (42) sur toute la longueur de l'arc lorsque le disque obturateur (12) est dans la position non obturée.

7. Valvule cardiaque prothétique selon la revendication 6, dans laquelle l'élément incurvé (27) est situé dans un plan aligné de façon à former un angle de 50° à 89° avec le plan de la bague de montage (11).

8. Valvule cardiaque prothétique selon la revendication 7, dans laquelle l'élément incurvé (27) est situé dans un plan aligné de façon à former un angle de sensiblement 75° avec le plan de la bague de montage (11).

9. Valvule cardiaque prothétique selon l'une quelconque des revendications précédentes, dans laquelle la forme et les dimensions de l'alvéole (42) et du moyen de guidage (21) en position proximale sont mutuellement complémentaires afin d'aider à empêcher l'accumulation de matière biologique indésirée à l'intérieur de l'alvéole (42) pendant l'utilisation.

10. Valvule cardiaque prothétique selon l'une quelconque des revendications précédentes, dans laquelle le moyen de guidage (22) en position distale comprend des première et seconde saillies (31, 32) de forme sensiblement en triangle, s'étendant chacune vers l'intérieur de la bague (11) de montage et ayant chacune une surface d'appui (34) destinée à coopérer avec la surface distale du disque obturateur (12).

FIG 1

FIG 2

1

FIG 3

FIG 4

FIG 5

FIG 6

FIG 7

FIG 8

FIG 9